# EUROPEAN PATENT SPECIFICATION

(11) **EP 1 765 997 B1**
(45) Date of publication and mention of the grant of the patent: **13.04.2016**
(21) Application number: 05745594.1
(22) Date of filing: 27.05.2005
(51) Int. Cl.: C12Q 1/34, C07K 16/40, C12N 9/20

(54) **MODULATING THE ACTIVITY OF TRIGLYCERIDE HYDROLASE**
MODULATION DER AKTIVITÄT VON TRIGLYCERID-HYDROLASE
MODULANT L'ACTIVITE D'UN TRIGLYCERIDE HYDROLASE

(30) Priority: 27.05.2004 AT 9242004
(43) Date of publication of application: 28.03.2007
(73) Proprietor: Karl-Franzens-Universität Graz, 8010 Graz (AT)
(72) Inventor: ZECHNER, Rudolf, A-8054 Graz (AT); ZIMMERMANN, Robert, A-8010 Graz (AT); STRAUSS, Juliane, G., A-8010 Graz (AT); HÄMMERLE, Günter, A-8010 Graz (AT); LASS, Achim, A-8010 Graz (AT)
(74) Representative: Vossius & Partner Patentanwälte Rechtsanwälte mbB
(86) International application number: PCT/EP2005/005710
(87) International publication number: WO 2005/115461

(56) References cited:
- DATABASE EMBL [Online] Sequence 9 from patent WO0107628 21 February 2001 (2001-02-21), TANG Y.T. ET AL.: "HUMAN SYNTHETASES" XP002361870 retrieved from EBI Database accession no. AX077778 & WO 01/07628 A (INCYTE GENOMICS, INC; TANG, Y., TOM; HILLMAN, JENNIFER, L; BANDMAN, OL) 1 February 2001 (2001-02-01)
- DATABASE EMBL [Online] Sequence 3 from patent WO2005014645 3 March 2005 (2005-03-03), BECERRA P.S. ET AL.: "Pedr-r receptor and uses" XP002361871 retrieved from EBI Database accession no. CS028979 & WO 2005/014645 A (THE GOVERNMENT OF THE UNITED STATES OF AMERICA, ASREPRESENTED BY THE S) 17 February 2005 (2005-02-17)
- ZIMMERMANN ROBERT ET AL: "Fat mobilization in adipose tissue is promoted by adipose triglyceride lipase" SCIENCE (WASHINGTON D C), vol. 306, no. 5700, 19 November 2004 (2004-11-19), pages 1383-1386, XP002359734 ISSN: 0036-8075
- VILLENA JOSEP A ET AL: "Desnutrin, an adipocyte gene encoding a novel patatin domain-containing protein, is induced by fasting and glucocorticoids - Ectopic expression of desnutrin increases triglyceride hydrolysis" JOURNAL OF BIOLOGICAL CHEMISTRY, vol. 279, no. 45, 5 November 2004 (2004-11-05), pages 47066-47075, XP002359735 ISSN: 0021-9258
- RABEN D M ET AL: "A new lipase in regulating lipid mobilization: hormone-sensitive lipase is not alone" TRENDS IN ENDOCRINOLOGY AND METABOLISM, ELSEVIER SCIENCE PUBLISHING, NEW YORK, NY, US, vol. 16, no. 2, March 2005 (2005-03), pages 35-36, XP004768091 ISSN: 1043-2760
- MILES J M ET AL: "Effect of orlistat in overweight and obese patients with type 2 diabetes treated with metformin", DIABETES CARE, AMERICAN DIABETES ASSOCIATION, ALEXANDRIA, VA, US, vol. 25, no. 7, 1 July 2002 (2002-07-01), pages 1123-1128, XP002294530, ISSN: 0149-5992
- P. A. HOLLANDER ET AL: 'Role of orlistat in the treatment of obese patients with type 2 diabetes. A 1-year randomized double-blind study' DIABETES CARE vol. 21, no. 8, 01 August 1998, pages 1288 - 1294, XP055035442 DOI: 10.2337/diacare.21.8.1288 ISSN: 0149-5992
- HOLLANDER P: "ORLISTAT IN THE TREATMENT OF OBESITY", PRIMARY CARE, SAUNDERS, LONDON, GB, vol. 30, no. 2, 1 January 2003 (2003-01-01), pages 427-440, XP001183450, ISSN: 0095-4543, DOI: 10.1016/S0095-4543(03)00042-3
- SMITH ET AL: "Multiple Risk Factors for Cardiovascular Disease and Diabetes Mellitus", AMERICAN JOURNAL OF MEDICINE, EXCERPTA MEDICA, INC, UNITED STATES, vol. 120, no. 3, 21 February 2007 (2007-02-21), pages S3-S11, XP005915951, ISSN: 0002-9343, DOI: 10.1016/J.AMJMED.2007.01.002
- Peter W. F. Wilson ET AL: "Prediction of Incident Diabetes Mellitus in Middle-aged Adults", ARCH INTERN MED, vol. 167, 2 May 2007 (2007-05-02), pages 1068-1074, XP055209108,

## Description

### FIELD OF THE INVENTION

The present invention provides an antibody as inhibitor of the activity of adipose triglyceride lipase encoded by a DNA sequence comprising the sequence according to SEQ ID NO: 1 for use in the treatment of obesity, type 2 diabetes or metabolic syndrome.

The present invention provides also a method for determining the triglyceride hydrolase activity of a protein comprising a polypeptide strand encoded by the DNA sequence according to SEQ No. 1 in an aqueous sample in presence of hormone sensitive lipase (HSL) characterized in that alkali metal halogenide is added to the sample in an amount effective to suppress the activity of said hormone sensitive lipase, whereafter the triglyceride hydrolase activity is determined.

### BACKGROUND OF THE INVENTION

Animals, seed plants, and fungi commonly store excessive amounts of energy substrates in the form of intracellular triglyceride (TG) deposits. In mammals, TG are stored in adipose tissue providing the primary source of energy during periods of food deprivation. Whole body energy homeostasis depends on the precisely regulated balance of lipid storage and mobilization. Mobilization of stored fat critically depends on the activation of lipolytic enzymes, which degrade adipose TG and release non-esterified fatty acids (FA) into the circulation. Dysregulation of TG-lipolysis in man has been linked to variation in the concentration of circulating FA, an established risk factor for the development of insulin resistance (1-4).

During periods of increased energy demand, lipolysis in adipocytes is activated by hormones, such as catecholamines. Hormone interaction with G-protein coupled receptors is followed by increased adenylate cyclase activity, increased cAMP levels, and the activation of cAMP-dependent protein kinase (protein kinase A, PKA) (5). PKA phosphorylates two important targets with established function in lipolysis: hormone-sensitive lipase (HSL), currently the only enzyme known to catabolize adipose tissue TG and perilipin A, an abundant protein located on the surface of lipid droplets. These modifications result in the translocation of HSL from the cytoplasma to the lipid droplet where efficient TG hydrolysis occurs (6).

Current models depict HSL as the rate-limiting enzyme in TG mobilization. However, recent observations of HSL knock-out (HSL-ko) mice are inconsistent with predictions of these models: HSL-deficient adipose tissue retains a marked basal and PKA-stimulated lipolytic capacity (7, 8) and HSL-ko mice exhibited normal body weight and were not obese. Instead, these animals exhibited reduced adipose tissue mass (9, 10) due to the

downregulation of triglyceride synthesis (10). The accumulation of diglycerides (DG) in various tissues of HSL-ko mice suggests that HSL is actually rate-limiting for the hydrolysis of DG in vivo but not for the catabolism of TG (7). These results imply the existence of one or more unidentified lipase(s) in adipose tissue that preferentially hydrolyze(s) the first ester bond (sn-1 or sn-3) of the TG molecule. WO 01/07628 discloses a protein having an amino acid sequence shown in SEQ ID NO: 9 therein, wherein said document does not disclose a function of the protein. Also an antibody specifically binding to the protein is disclosed. Miles (Diabetes care 25(7): 1123-1128 (2002)), Hollander (Diabetes care 21(8): 1288-1294 (1998)) and Hollander (Prim Care Clin Office Pract 30: 427-440 (2003)) disclose the use of the lipase inhibitor orlistat in the therapy of obesity as well as of overweight and obese patients with type 2 diabetes.

### SUMMARY OF THE INVENTION

Herein it is disclosed that a lipase expressed in adipose tissue that fulfills the requirements for an enzymatically active TG-hydrolase also is expressed at high levels in murine adipose tissue. For the purpose of the present specification the lipase is termed "adipose triglyceride lipase" (ATGL). ATGL has triglyceride hydrolase activity.

The DNA coding for the lipase comprises the sequence according to SEQ No. 1. This sequence is identical to the coding sequence 203-1717 of NCBI nucleotide entry NM_020376 (gi: 34147340). Modulating the activity of ATGL affects the liberation of free fatty acids from adipose tissue and consequently the plasma level of free fatty acids, triglycerides and glucose. Modulating the liberation of free fatty acids from adipose tissue is desirable in disorders like obesity, type 2 diabetes and metabolic syndrom.

The activity of ATGL can be modulated by means of inhibitors or activators. An activator useful to enhance ATGL activity is CGI-58 (comparative gene identification 58) as described herein. Accordingly, the present invention relates to the use of CGI-58 (Comparative gene identification 58) as activator of a protein comprising a polypeptide strand encoded by the DNA sequence according to SEQ No. 1 *in vitro*. It is found herein that known lipase inhibitors and antibodies may be useful as inhibitors against ATGL. The invention is therefor directed to an antibody as inhibitor of the activity of adipose triglyceride lipase encoded by a DNA sequence comprising the sequence according to SEQ No. 1 for use in the treatment of obesity, type 2 diabetes or metabolic syndrome.

The invention is also directed to a method for determining the triglyceride hydrolase activity of a protein comprising a polypeptide strand encoded by the DNA sequence according to SEQ No. 1 in an aqueous sample in presence of hormone sensitive lipase (HSL), characterized in that alkali metal halogenide is added to the sample in an amount effective to suppress the activity of said hormone sensitive lipase, whereafter the triglyceride hydrolase activity of ATGL is determined. It has turned out that an alkali metal halogenide can selectively suppress the activity of HSL.
In a preferred embodiment of the method according to the invention said alkali metal halogenide is potassium chloride.

These methods are therefore useful diagnostic tools to determine ATGL activity in plasma or any other body fluid.

### DETAILED DESCRIPTION OF THE INVENTION

The following experimental part was undertaken with mouse ATGL, the cDNA of which exhibiting more than 96% homology to human DNA coding for human ATGL.

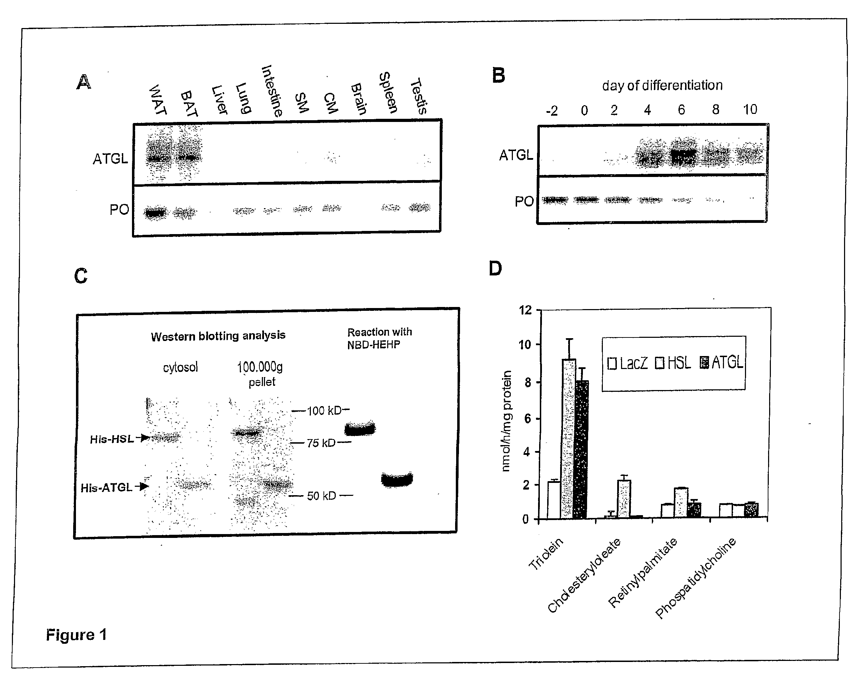

The full length cDNA of ATGL containing the complete ORF was amplified by RT-PCR from total RNA of mouse white adipose tissue and subjected to DNA sequence determination. The nucleotide sequence of mouse ATGL is shown as SEQ No. 2 and exhibits 100% sequence identity to NCBI nucleotide entry AK031609 (gi: 26327464). The 1.460 bp coding sequence specifies a putative protein of 486 amino acids (NCBI accession number BAC27476) with a calculated molecular weight of 53.652 D. Northern blotting analysis of total RNA from various C57B16 mouse tissues revealed that ATGL mRNA is expressed at high levels in white and brown adipose tissue (Figure 1A). Weak mRNA signals for ATGL were additionally observed in testis, cardiac muscle and skeletal muscle. During a differentiation time course of murine 3T3-L1 adipocytes, ATGL mRNA expression was first detected 4 days after induction of differentiation and a maximum of expression was obtained at day 6 (Figure 1B). This mRNA expression profile is typical for late markers of adipocyte differentiation and closely resembles the expression pattern of HSL mRNA (not shown).

To investigate whether ATGL hydrolyzes neutral lipids, His-tagged ATGL was transiently expressed in COS-7 cells using an eukaryotic expression vector. For comparison, COS-7 cells were also transfected with a similar construction expressing His-tagged HSL. Both His-tagged ATGL and HSL protein were detected in the cytosolic supernatant and the membrane pellet fraction of transfected COS cells by Western blotting analysis (Figure 1C). The apparent molecular weights of ATGL and HSL were estimated as 54 kD and 84 kD, respectively. When extracts from transfected cells were preincubated with a fluorescent lipase inhibitor (NBD-HEHP) (11) and subsequently subjected to SDS-PAGE analysis and fluorography, fluorescent signals were observed in positions corresponding to the expected molecular weight of ATGL and HSL (Figure 1C). The fact that the fluorescent probe only reacts with enzymatically active Ser-lipases (11) provided evidence that ATGL is enzymatically active in transfected COS cells. To confirm this, TG-hydrolase activity assays were performed using a radioactively labelled [9,10-3H(N))]-triolein substrate (Figure ID).

The cytosolic fractions of ATGL transfected COS-7 cells exhibited a marked increase in TG hydrolase activity (3.7-fold compared to LacZ transfected control cells). No enzymatic activities were observed when radioactively labeled retinyl palmitate, cholesteryl oleate or phosphatidylcholine were used as lipid substrates. In accordance with previous data (12, 13), cytosolic fractions of HSL-transfected cells exhibited increased TG hydrolase (4.2-fold), cholesteryl ester hydrolase (23-fold), and retinyl-ester hydrolase (2.3-fold) activities compared to lacZ transfected cells. Thus ATGL possesses triglyceride hydrolase activity, but in contrast to HSL, this enzyme appears to be substrate-specific for TG and does not hydrolyze cholesteryl- or retinyl-ester bonds.

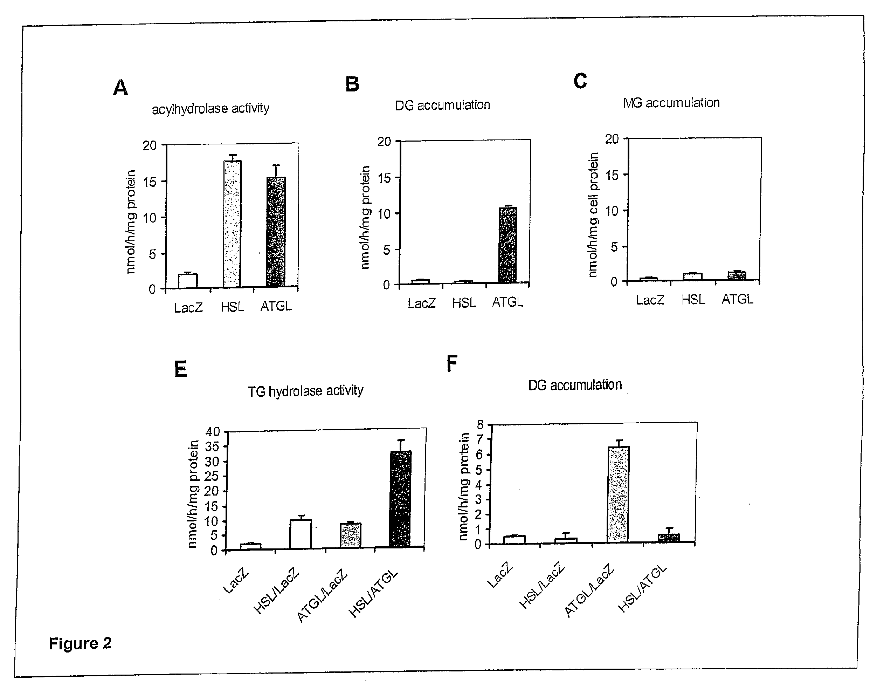

To specify the function of ATGL in TG catabolism in comparison to HSL, the relative abundance of lipolytic reaction products after incubation of a [9,10-3H(N)]-triolein labeled substrate with cytosolic extracts of ATGL or HSL transfected COS-7 cells was determined. Reaction products were separated by TLC and quantitated via scintillation counting of distinct lipid fractions (Figure 2). Compared to control extracts of LacZ transfected cells, extracts from ATGL and HSL-transfected cells contained 7.5 and 10-fold higher activities, respectively (Figure 2A). In the presence of ATGL the accumulation of diacylglycerol (DG) was increased 21-fold compared to LacZ transfected cells suggesting that the enzyme predominantly hydrolyzed the first ester bond of TG (Figure 2B). TLC analysis of DG isomers indicated a strong preference of ATGL for the sn-1 position of TG (not shown). In contrast, lipolysis assays with cytosolic extracts from HSL transfected cells did not result in DG accumulation. The finding of efficient cleavage of DG by HSL observed here is consistent with the previously observed high substrate specificity of HSL for DG (10-fold higher than for TG) (14). Monoglyceride (MG) accumulation was only barely detectable with extracts of ATGL and HSL transfected cells (Figure 2C). From the molar ratios of DG and MG accumulation vs. FA release it can be calculated that ∼90% of the FA molecules released in the presence of ATGL originate from the hydrolysis of TG in the first ester bond. In contrast, in the presence of HSL, most FA originate from all three ester bonds resulting in glycerol formation. Thus, the results demonstrate that ATGL and HSL possess distinctly different substrate-specificities within the lipolytic cascade, suggesting that they might act coordinately in the catabolism of TG.

This assumption was confirmed by the product profiles generated in triolein hydrolysis assays using the combined extracts of LacZ, ATGL, or HSL transfected cells (Figure 2E). Relative to extracts from LacZ transfected cells, the acyl-hydrolase activity was increased in equal volume mixtures of HSL/LacZ extracts (4.8-fold), ATGL/LacZ extracts (4-fold) and ATGL/HSL extracts (16-fold). The accumulation of DG was increased 12.5-fold when LacZ/ATGL extracts were used and reduced to basal levels with ATGL/HSL extracts (Figure 2F).

Without being bound by theory, considering this marked difference in substrate specificity of ATGL and HSL, it is thought that during the lipolytic breakdown of TG, ATGL is predominantly responsible for the initial step of TG hydrolysis whereas HSL acts to hydrolyze the resulting DG to monoglycerides. These, in turn, are converted to FA and glycerol by monoglyceride lipase (15). This model is supported by a marked cooperative effect observed in the combined presence of ATGL and HSL. As shown in Figure 2E, the total acyl-hydrolase activity in ATGL/HSL containing extracts was nearly 2-fold higher than the sum of the individual activities.

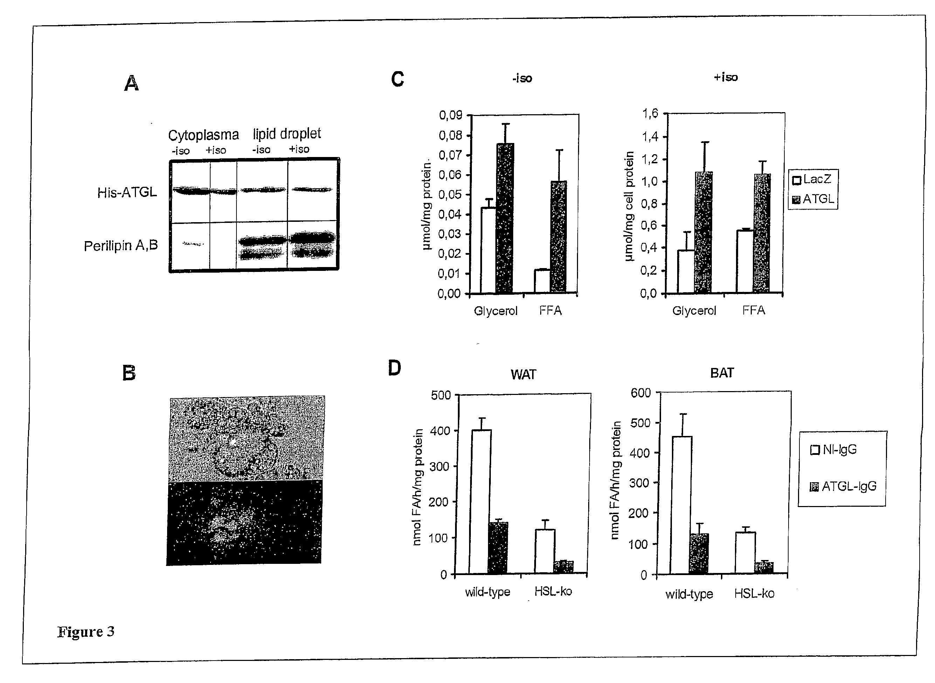

To determine whether ATGL is functional also in adipocytes, a recombinant adenovirus encoding the His-tagged full length mouse ATGL cDNA was constructed and used to infect mouse 3T3-L1 adipocytes at day 6 of differentiation. Western blotting analysis of cell-extracts of infected adipocytes revealed expression of His-tagged ATGL at the appropriate molecular weight (Figure 3A). The enzyme was found to be tightly associated with lipid droplets of adipocytes even after extensive purification of the droplets by multiple centrifugation (16). Stimulation of lipolysis by isoproterenol did not affect the localization of the enzyme arguing for a constitutive association of ATGL with lipid droplets in adipocytes. Additionally, ATGL expressing 3T3-L1 cells released higher levels of FA (5-fold) and glycerol (1.8-fold) compared to LacZ infected cells under basal conditions. After isoproterenol stimulation, FA release was increased by 1.8-fold and glycerol release by 2.9-fold compared to LacZ expressing control cells. Thus, overexpression of ATGL in adipocytes can markedly augment both basal and isoproterenol-stimulated lipolysis, indicative for a functional lipase in adipose tissue.

In summary, ATGL is a potent TG hydrolase with little or no specificity for DG, cholesteryl ester, retinyl ester and phosphatitylcholine. The mouse enzyme is predominantly expressed in adipose tissue. It is lipid droplet associated and enhances basal and β-adrenergically stimulated FA release. Although the regulatory mechanism for the activation of ATGL remain to be elucidated, these findings suggest that the enzyme is an important component of the lipolytic process and the mobilization of lipid stores in mammals.

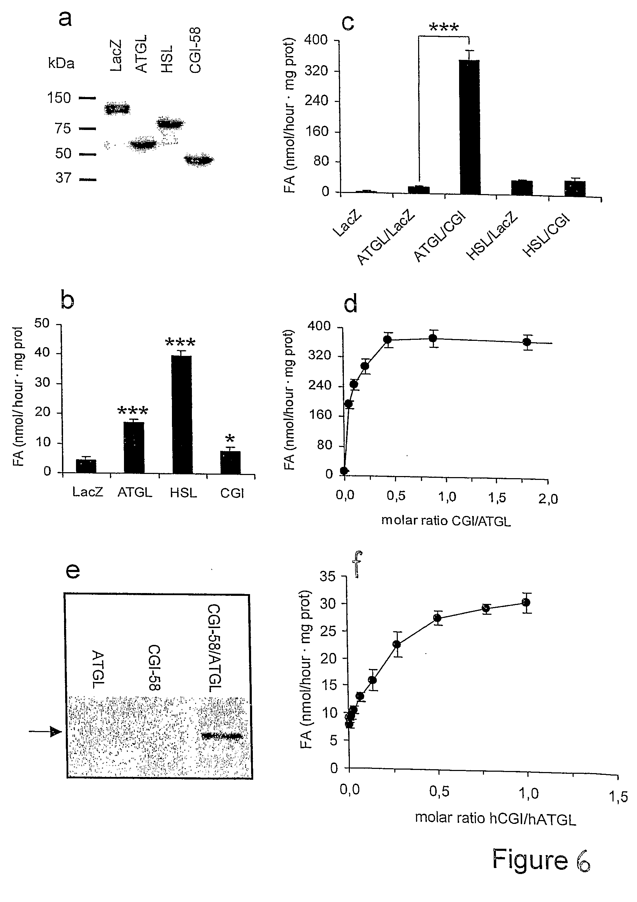

Also the suitability of CGI-58, a gene encoding a lipid droplet associated protein with unknown function as an activator of ATGL was studied. Said gene was found to exhibit mutations in subjects suffering from the Chanarin-Dorfman Syndrome (CDS), which is a rare autosomal recessive disorder characterized by intracellular accumulation of triglycerides in multiple vacuoles in most tissues and blood granulocytes. In order to investigate whether CGI-58 is able to affect cellular TGH activity in a comparable manner with ATGL or HSL, simian virus-40 transformed monkey kidney cells (COS-7) were transfected with cDNA clones expressing His-tagged murine CGI-58, ATGL, HSL or LacZ as a control. Expression of respective proteins in COS-7 was confirmed by Western blotting (Fig. 6a) and cytoplasmic extracts of the transfected cells where subjected to TG hydrolase assay. As shown in Fig. 6b, expression of CGI-58 increased the TGH activity by 76% compared to LacZ transfected cells. In comparison, transfection of cells with ATGL and HSL increased TGH activities 4- and 9-fold, respectively. In order to investigate whether the effect of CGI-58 is due to endogenous TGH activity of CGI-58 or if the protein affects the activity of other lipases, the extracts of CGI-58 and ATGL or HSL-expressing cells where mixed together and subjected to TGH activity determinations (Fig. 6c). In the presence of ATGL and CGI-58, TG-hydrolase activity was enhanced 80-fold compared to the LacZ control, indicating that CGI-58 substantially increases the activity of ATGL. In contrast, CGI-58 had no effect on the activity of hormone-sensitive lipase which suggests that the protein specifically activates ATGL (Fig. 6c). A dose-response experiment revealed that maximal ATGL activity was achieved at a molar CGI-58/ATGL ratio of approximately 0.5 (fig. 6d). The activation of ATGL by CGI-58 could also be monitored on the molecular level using the fluorescently labeled lipase-inhibitor NBD-sn1TG. By mimicking a TG molecule, this inhibitor covalently binds to active lipases. As shown in Fig. 6e, in the presence of CGI-58 the fluorescent signal for ATGL in cytoplasmic extracts was intensified ~5-fold. Thus, the results suggest that CGI-58 is capable of increasing the cellular TGH activity by activation of ATGL.

To compare the activities of human and murine proteins, human CGI-58 (hCGI-58) and human ATGL (hATGL) were expressed in COS-7 cells and tested in TGH activity assay (Fig. 6f). Similarly as shown for the mouse proteins, hCGI-58 increased the activity of hATGL in a dose dependent manner. In comparison to the mouse orthologes (Fig. 6d), the magnitude of the maximal effect on ATGL activation was smaller (6-fold versus 20-fold) suggesting species-dependent differences in the specific activities of human and mouse proteins.

In summary, the study on CGI-58 provides evidence that CGI-58 acts as activator of ATGL and is therefore able to enhance the cellular capacity to mobilize free fatty acids from the TG pool.

### Material and Methods

*cDNA cloning and transient expression of recombinant His-tagged proteins in COS-7 cells and 3T3-L1 adipocytes*. The coding sequences of ATGL and HSL were amplified by PCR from cDNA prepared from mRNA of mouse white adipose tissue by reverse transcription. The open reading frame, flanked by *Kpn*Il*Xho*I sites for ATGL and HSL were cloned into the eucaryotic expression vector pcDNA4/HisMax (Invitrogen). Transfection of COS-7 cells was performed with Metafectene™ (Biontex) according to the manufacturer's description. The PCR primers used to generate these probes were as follows.
ATGL forward 5'-*TGGTACCG*TTCCCGAGGGAGACCAAGTGGA-3',
ATGL revers 5'-*CCTCGAGC*GCAAGGCGGGAGGCCAGGT-3'.
HSL forward *5'-TGGTACCT*-ATGGATTTACGCACGATGACACA-3',
HSL revers 5'-*CCTCGAGC*GTTCAGTGGTGCAGCAGGCG-3'.

*cDNA cloning of recombinant His-tagged proteins for CGI-58 investigations -* Total RNA was isolated from mouse and human adipose tissue using the Trizol® Reagent procedure according to the manufacturer's instruction (Invitrogen life technologies, Carlsbad, CA). Poly A⁺ RNA was isolated from total RNA using the Oligotex® mRNA Mini Kit from Qiagen GmbH (Hilden, Germany). mRNA was transcribed into first-strand cDNA using SuperScript™ Reverse Transcriptase protocol from Invitrogen life technologies. Second-strand cDNA was obtained by addition of E. coli DNA ligase buffer, E. coli DNA polymerase, E. coli DNA ligase (all chemicals from New England Biolabs Inc., Beverly, MA), and dNTPs (Carl Roth GmbH & Co. KG, Karlsruhe, Germany) to the mixture and subsequent incubation at 16°C for 3 h. Thereafter, T4 DNA polymerase (New England Biolabs Inc.) was added and further incubated for 20 min to give blunt end cDNA. The coding sequences of mouse ATGL, HSL, CGI-58, and human ATGL (TTS-2.2) were amplified by PCR from mouse and human adipose tissue cDNA using Advantage® cDNA Polymerase Mix (BD Biosciences Clontech, Palo Alto, CA), respectively. The primers were designed to create KpnI (5') and XhoI (3') restriction endonuclease cleavage sites for mouse ATGL and HSL and BamHI (5') and XhoI (3') sites for human ATGL:
mouse ATGL forward 5'-TGGTACCGTTCCCGAGGGAGACCAAGTGGA-3',
mouse ATGL reverse 5'-CCTCGAGCGCAAGGCGGGAGGCCAGGT-3',
mouse HSL forward 5'-TGGTACCTATGGATTTACGCACGATGACACA-3',
mouse HSL reverse 5'-CTCGAGCGTTCAGTGGTGCAGCAGGCG-3',
mouse CGI-58 forward 5'- -3',CGGATCCAAAGCGATGGCGGCGGAGGA,
mouse CGI-58 reverse 5'- -3',CCTCGAGTCAGTCTACTGTGTGGCAGATCTCC,
human ATGL forward 5'-CGGGATCCTTTCCCCGCGAGAAGACGTG-3',
human ATGL reverse 5'-CCCTCGAGCTCACAGCCCCAGGGCCCC-3',
The PCR products, containing the complete open reading frame, were ligated to compatible restriction sites of the eukaryotic expression vector pcDNA4/HisMax (Invitrogen life technologies). A control pcDNA4/HisMax vector expressing β-galactosidase (LacZ) was provided by the manufacturer (Invitrogen life technologies).

*Construction of the recombinant adenovirus for ATGL expression (ATGL-Ad) and infection of 3T3-L1 cells:* The recombinant adenovirus coding for mouse ATGL was prepared by cotransfection of the shuttle plasmid pAvCvSv containing the ATGL cDNA and pJM 17 into HEK-293 cells. The 1.65 kb Mlu I - Cla I flanked mouse ATGL cDNA fragment (His-tag included) was amplified by PCR from the eucaryotic expression vector pcDNA4/HisMax containing mouse ATGL cDNA and subcloned into Mlu I - Cla I digested pAvCvSv. The resulting shuttle plasmid was cotransfected with pJM 17 into HEK-293 cells using the calcium phosphate coprecipitation method. Large scale production of high titer recombinant ATGL-Ad was performed as described elsewhere. 3T3-L1 fibroblasts were cultured in DMEM containing 10% FCS and differentiated using a standard protocol (27). Adipocytes were infected on day 8 of differentiation with a multiplicity of infection (moi) of ~400 plaque forming units/cell. For that purpose appropriate pfu were preactivated in DMEM containing 0.5 µg/ml of polylysin for 100 min and afterwards the cells were incubated with this virus suspension for 24 hours. After 24 h the medium was removed and the cells were incubated for further 24 h with complete medium. For most of the experiments, recombinant adenovirus expressing ß-galactosidase was used as a control (LacZ-Ad).

*Expression of recombinant proteins in cultured cells for CGI-58 investigations -* Monkey embryonic kidney cells (COS-7, ATCC CRL-1651) were maintained in Dulbecco's minimal essential medium (DMEM) (Gibco, Invitrogen life technologies, Carlsbad, CA) containing 10% fetal calf serum (FCS) (Sigma-Aldrich Chemie GmbH) and antibiotics at 37°C in humidified air (89-91% saturation) and 5% CO₂. The day before transfection COS-7 cells were collected in logarithmic phase, seeded in 6-wells dishes at a density of 150,000 cells/well and cultured overnight. Transient transfection of COS-7 cells with pcDNA4/HisMax vector coding His-tagged proteins was performed with Metafectene™ (Biontex GmbH, Munich, Germany). One to two µg purified plasmid DNA (NucleoBond® AX, Macherey-Nagel GmbH &Co. KG, Düren, Germany) were mixed with 5µl Metafectene in a total volume of 100 µl serum and antibiotics-free DMEM and incubated for 20 min at RT to allow formation of the DNA/Metafectene complex. Then, 100 µl/well of the DNA/Metafecetene mix were added and incubated for 4 hours in serum and antibiotics-free DMEM. Thereafter, the medium was removed and cells were cultured in DMEM containing 10 % FCS and antibiotics. Cells were analyzed two days after transfection.

*Subcellular fractionation of COS-7 cells.* Transfected COS-7 cells were collected by trypsinisation and washed three times with PBS. Cells were disrupted on ice in lysis buffer (0.25 M sucrose, 1 mM EDTA, 1 mM dithiothreitol, 20 µg/ml leupeptin, 2 µg/ml antipain, 1 µg/ml pepstatin, pH 7) by sonication (Virsonic 475). Nuclei and unbroken materials were removed by centrifugation at 1.000 g at 4°C for 15 min to obtain cytoplasmatic extracts. The cytplasmatic extracts were centrifuged at 100.000g at 4°C for one hour to obtain cytosolic extracts and membrane pellets.

*Isolation of lipid droplets.* 3T3-L1 adipocytes from two 10 cm plates were disrupted in buffer A (20 mM Tricine, pH 7.8, 0.25 M sucrose, 2 mM MgCl₂ 0.2 mM PMSF) by sonication (Virsonic 475). 6 ml of puffer A were overlaid with 6 ml of buffer B containing 20 mM Hepes (pH 7.4), 100 mM KCl, 2 mM MgCl₂, 0,2 mM PMSF and centrifuged for 3 hours at 40.000 rpm at 4°C. The lipid droplets concentrating at the top of the tube were collected and washed several times with buffer B as described (28).

*Western analysis*. Cellular proteins were separated by SDS-polyacrylamide gel electrophoresis and transferred to a nitrocellulose membrane (Schleicher & Schuell, Germany). For detection of His-tagged proteins, blots were incubated with 1/10000 diluted Anti-His monoclonal antibody (6xHis, Clonetech). Perilipin was detected using a guinea pig polyclonal antibody against Perilipin A and B (PROGEN). Bound immunoglobulins were detected with a HRP-labeled IgG conjugates (Vector Inc.) and and visualized by ECL detection (ECL plus, Amersham Pharmacia Biotech, Germany) on a Storm Image Analysis system. Quantitation was performed using ImageQuant Software.

*Western blot analysis for CGI-58 investigations -* Transfected COS-7 cells were solubilized in SDS-PAGE sample puffer, cell proteins were separated on a 10 % SDS-PAGE gel using the Laemmli discontinuous buffer system (ref) and transferred onto a polyvinylidene fluoride transfer membrane (Pall Life Sciences, Pensacola, FL). The membrane was blocked with 2% blotting grade milk powder (Carl Roth GmbH & Co.) in Tris/NaCl/Tween 20 and incubated with mouse anti-His monoclonal antibody (6xHis, Amersham Biosciences Corp., Piscataway, NJ) at a dilution of 1:7,000. The blots were washed 3 times in Tris/NaCl/Tween 20 for 10 min; after incubation with horseradish peroxidase-conjugated sheep anti-mouse (Amersham Biosciences Corp.) at a dilution of 1:10,000, the membranes were developed with enhanced chemiluminescence (ECL plus, Amersham Biosciences Corp.) and exposed to x-ray film (Hyperfilm™ ECL, Amersham Bioscience Corp.).

*Reaction of ATGL and HSL with the fluorescent lipase inhibitor NBD*-*HEHP*. Transfected COS-7 cells were washed twice with PBS, scraped into lysis buffer (0.25 M sucrose, 1 mM EDTA, 1 mM dithioerythritol, 20 µg/ml leupeptin, 2 µg/ml antipain, 1 µg/ml pepstatin) and disrupted on ice by sonication. Nuclei and unbroken materials were removed by centrifugation at 1.000 g at 4°C for 15 min to obtain cytoplasmatic extracts. 50 µg of protein was incubated with 1 nmol fluorescently labelled lipase inhibitor O-((6-(7-Nitrobenz-2-oxa-1,3-diazol-4-yl)amino)hexanoyl)aminoethyl-O-(n-hexyl)phosphonic acid p-nitrophenyl ester (NBD-HEHP) (29) and 1 mM Triton X-100 (especially purified for membrane research, Hofmann LaRoche) at 37°C for 2 hours under shaking. Protein was precipitated with 10% TCA for 1h on ice, washed with acetone and separated by 10% SDS-PAGE. Gels were fixed in 10% ethanol and 7% acetic acid. Fluorescence was detected with a BioRad FX Pro Laserscanner (excitation 488 nm, emission 530 nm).

*Northern analysis*. The cDNA probe for northern blot analysis of mouse ATGL was prepared by RT-PCR by use of first-strand cDNA from mouse fat mRNA. The PCR primers used to generate this probe were as follows: forward 5'-TGGAACATCTCATTCGCTGG-3', revers 5'-AATGCCGCCATCCACATAG-3'. Total RNA was isolated from various mouse tissues using the TRI Reagent procedure according to manufacturer's protocol (Molecular Research Center, Karlsruhe, Germany). Specific mRNAs were detected using standard Northern blotting techniques with 10 µg total RNA. ³²P-labeled probes for hybridization were generated using random priming. Northern blots were visualized by exposure to a PhosphorImager Screen (Apbiotech, Freiburg, Germany) and analyzed using ImageQuant Software.

*Assay for TG lipase, cholesteryl esterase, retinyl esterase and phospholipase activity*. For determination of lipase activity 0.1 ml of cytosolic extracts and 0.1 ml substrate were incubated in a water bath at 37 °C for 60 min. The reaction was terminated by adding 3.25 ml of methanol/chloroform/heptane (10:9:7) and 1 ml of 0.1 M potassium carbonate, 0.1 M boric acid, pH 10.5. After centrifugation (800 g, 20 min) the radioactivity in 1 ml of the upper phase was determined by liquid scintillation counting. Neutral lipase activity was measured in 50 mM potassium phosphate buffer, pH 7.0 and 2.5% defatted BSA. The substrate for neutral TG lipase activity contained 33 nmol triolein/assay with [9,10-³H(N)]-triolein (40.000 cpm/nmol, NEN Life Science Products) as radioactive tracer for COS-7 cells and 167 nmol/assay for 3T3-L1 adipocytes (7300 cpm/nmol). The substrates for cholesteryl esterase and retinyl esterase activity contained 10 nmol/assay of cholesteryl oleate or retinyl palmitate and the corresponding tracers cholesteryl [9,10-³H]-oleate or retinyl [9,10-³H(N)]-palmitate (50.000 cpm/nmol). For determination of phospholipase activity in cytosolic extracts the substrate contained 20 nmol/assay phosphatidylcholine and [dipalmitoyl-1-¹⁴C]-phosphatidylcholine (12.000 cpm/nmol). All substrates were prepared by sonication (Virsonic 475) essantially as described (30).

For investigation of DG formation in the *in vitro* assay the reaction was terminated by adding 1 ml of CHCl₃/Methanol (2:1) containing oleic acid (10 µg/ml) and standards for mono- and dioleine (sn-1.2 and sn-1.3; Sigma). The mixture was vortexed vigorously three times over a period of 15 min. After centrifugation (4000 g, 10 min), 0.5 ml of the lower phase was collected and evaporated under nitrogen. The lipid pellet was dissolved in chloroform and loaded onto a TLC plate (Merck Silica gel 60). The TLC was developed with chloroform/acetone/acetic acid (96:4:1) as solvent. The lipids were visualized with iodine vapor and the bands corresponding to mono-, di-, trioleine and oleic acid were cut out. The comigrating radioactivity was determined by liquid scintillation counting.

*Determination of FA and glycerol release from 3T3*-*L1 adipocytes*. Cells were incubated in DMEM medium (GIBCO) containing 2% fatty acid free BSA (Sigma) with or without 10 µM isoproterenol (Sigma) at 37 °C. Aliquots of the medium were collected and investigated for the FFA and glycerol content by using commercial kits (WAKO).

### Detailed description of Figures 1-3

Figure 1. Northern blot analysis of ATGL mRNA expression in various mouse tissues and (A) during adipocyte conversion of 3T3-L1 cells (B) . 10µg of total RNA from fasted mice or 3T3 cells were subjected to Northern blot analysis and detected with a specific ³²P-labeled ATGL DNA probe. The acidic ribosomal protein PO was used as a control. 3T3-L1 cells were induced to differentiate into adipocytes two days after confluence (day 0) using a standard differentiation protocol (24). (C) Western blot analysis of His-tagged ATGL and HSL and reaction of the proteins with the fluorescent lipase inhibitor NBD-HEHP. Transient transfection of COS-7 cells was performed using the eukaryotic expression vector pcDNA4/HisMax (Invitrogen) coding for His-tagged full-length cDNA of ATGL or HSL. The His-tagged proteins were detected by immunoblotting in cytosolic extracts (100.000g supernatant) and in the membrane fraction (100.000g pellet). Blots were incubated with Anti-His monoclonal antibody and HRP-anti-mouse IgG conjugate and visualized by ECL detection. For the reaction with NBD-HEHP, cytoplasmic extracts were incubated with 1 nmol fluorescently labeled lipase inhibitor and 1 mM Triton X-100 at 37°C for 2 hours under shaking. Subsequently, the samples were subjected to SDS-PAGE and labeled proteins were visualized by a BioRad FX Pro Laserscanner. (D) Enzymatic activity and substrate specifity of ATGL. Cytosolic extracts of COS-7 cells expressing His-tagged ATGL, HSL or □-galactosidase (LacZ) were assayed for lipase activity using substrates containing radiolabeled triolein, cholesteryl oleate, retinyl palmitate or phosphatitylcholine. Experiments were performed in triplicate. Data are presented as mean ± S.D. and are representative for at least three independent experiments.
Figure 2. Role of ATGL within the triglyceride hydrolysis cascade. Cytosolic extracts of COS-7 cells, transiently transfected with His-tagged LacZ, ATGL or HSL, were incubated with triolein containing [9,10-³H(N)]-triolein as radioactive tracer. Lipids were extracted and separated by TLC using CHCl₃/aceton/acetic acid (96/4/1) as mobile phase. Lipids were visualized with iodine vapor and the radioactivity comigrating with MG, DG, TG and FA standards was determined by liquid scintillation counting. (A) Total acyl-hydrolase activity (FA). (B) Accumulation of DG. (C) Accumulation of MG. (D) Effect of combined activity of ATGL and HSL on TG hydrolase activity. Cytosolic extracts of COS cells expressing LacZ were mixed 1:1 with extracts from cells expressing ATGL or HSL (ATGL/LacZ and HSL/LacZ) and compared to extracts prepared from a mixture of ATGL and HSL expressing cells (ATGL/HSL). (E) Effect of combined activity of ATGL and HSL on DG accumulation. All experiments were performed in triplicate. Data are presented as mean ± S.D. and are representative for three independent experiments.
Figure 3. Cellular localization, lipolytic activity and antibody-directed inhibition of ATGL in adipocytes. (A) A recombinant adenovirus coding for His-tagged ATGL (ATGL-Ad) was used to infect adipocytes on day 8 after induction of differentiation and experiments were performed 2 days after infection. (16). Cells were cultured in DMEM medium (GIBCO) containing 2% fatty acid free BSA (Sigma) in the absence or in the presence of isoproterenol (10 µM at 37°C for two hours) as indicated (+ iso) prior to harvesting cells or medium. Western blot analysis of ATGL in the cytoplasmic fraction (10 µg of total protein) and in isolated lipid droplets (2 µg of total protein) of adipocytes using an anti-His monoclonal antibody. Purification of lipid droplets was monitored by the enrichment of perilipin (>70-fold) using a rabbit polyclonal antibody against perilipin A and B (Progen). (B) Fluorescent photograph of 3T3-L1 adipocytes transfected with GFP-ATGL. GFP-ATGL was introduced transiently in cells on day 8 after induction of differentiation and photographs were taken 2 days after infection. (C) Glycerol and FA release from ATGL-Ad infected adipocytes were measured in aliquots of culture medium using commercially available kits (WAKO). Recombinant adenovirus expressing ß-galactosidase (LacZ) was used as a control. Experiments were performed in triplicate. Data are presented as mean ± S.D. and are representative for three experiments. (D) Inhibiton of cytosolic acyl hydrolase activity in WAT and BAT by a polyclonal antibody against mouse ATGL (ATGL-IgG) using [9,10-³H(N)]-labeled triolein as substrate. The activity in cytosolic extracts of wild-type and HSL-ko mice was determined either in the presence of rabbit non-immune IgG (NI-IgG) or ATGL-IgG. Data are presented as mean ± S.D. of three single mice for each group and are representative for two experiments.

### Generation of a rabbit polyclonal antibody to murine ATGL

The recombinant adenoviral vector containing His-tagged cDNA was used to immunize a rabbit. Viral particles (5 x 10⁹ pfu/kg) were injected into a rabbit through the ear vein. Sera were obtained initially 6 weeks after infection and subsequently in intervals of 2 weeks for analysis of antibody reactivity in TG hydrolase assays and Western blotting experiments. The serum of a non-immunized rabbit was used as a control. The IgG fractions were isolated from rabbit serum using a protein G column (Amersham Pharmacia Biotech) according to the manufacturer's protocol.

### Determination of TG hydrolase activity

Neutral TG lipase activity was measured with triolein as substrate containing [9,10-3H(N)]-triolein (NEN Life Science Products) as radioactive tracer. The substrate for TG lipase activity was prepared by sonication (Virsonic 475) exactly as describeded by Holm *et al.* (30). Cells were disrupted on ice in lysis buffer (0.25 M sucrose, 1 mM EDTA, 1 mM dithiothreitol, 20 µg/ml leupeptin, 2 µg/ml antipain, 1 µg/ml pepstatin, pH 7) by sonication (Virsonic 475). The cytosolic infranatants were obtained after centrifugation at 1000,000 g, at 4 °C for 60 min. The reaction was performed in a water bath at 37 °C for 60 min with 0.1 ml substrate and 0.1 ml infranatant. The reaction was terminated by adding 3.25 ml of methanol/chloroform/heptane (10:9:7) and 1 ml of 0.1 M potassium carbonate, 0.1 M boric acid, pH 10.5. After centrifugation (800 g, 20 min) the radioactivity in 1 ml of the upper phase was determined by liquid scintillation counting.

### Effect of an inhibitor on ATGL activity

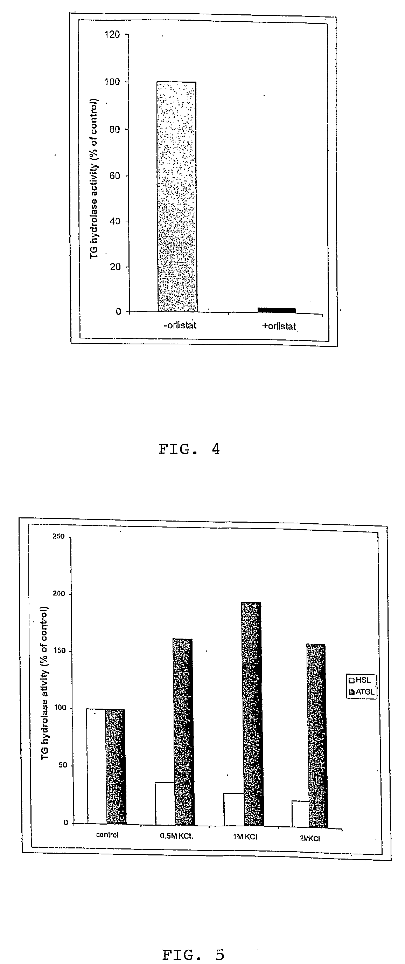

Figur 4 shows the effect of the known HSL inibitor orlistat (Xenical^{®}, Roche) on ATGL activity. A recombinant adenovirus coding for His-tagged ATGL or HSL was used to infect HepG2 cells as described above. For activity assays, the cytosolic fractions of the cells were incubated with a substrate containing radiolabeled triolein in the absence (control) or in the presence of 50µg/ml orlistat. It can be seen from Fig. 4 that addition of orlistat decreased in ATGL activity by 98%.

### Effect of alkali metal halogenide on HSL and ATGL activity

A recombinant adenovirus coding for His-tagged ATGL or HSL was used to infect HepG2 cells. The infection led to a 7- and 12-fold increase in TG hydrolase activity for HSL and ATGL, respectively, compared to LacZ-infected cells. For activity assays, the cytosolic fractions of the cells were incubated with a substrate containing radiolabeled triolein in the absence (control) or in the presence of the indicated salt concentrations.

The results are shown in Figure 5: Addition of KCl resulted in a dose dependent decrease in HSL activity (-68% at 1M KCl). In contrast, the activity of ATGL was stimulated by KCl (+84% at 1M KCl).

Figure 6. CGI-58 specifically activates ATGL TGH activity. Murine ATGL, HSL, and CGI-58 were cloned into His-tag pcDNA4/HisMax expression vector and recombinant proteins were transiently expressed in COS-7 cells. β-galactosidase (LacZ) was used as a control. (**a**) His-tagged proteins were detected in cytoplasmic extracts of transfected cells by Western blotting using a monoclonal anti-His antibody. (**b**) TGH activity of cytoplasmic extracts of transfected cells was determined using a radiolabeled triolein substrate. (**c**) Cytoplasmic extracts of cells expressing ATGL or HSL were mixed with extracts containing either CGI-58 or LacZ and TGH activity determined. LacZ was used as a control. (**d**) Dose-dependent effect of CGI-58 on ATGL TGH activity. Cytoplasmatic extracts of ATGL expressing cells were mixed with increasing concentrations of CGI-58 expressing extract and subjected to TGH activity assays. Expression levels of ATGL and CGI-58 in cytoplasmic extracts were visualized by Western blotting using anti-His antibody and quantitated densitometrically. Molar ratios were calculated by adjusting for intensity of expression of the respective His-tagged recombinant protein. (**e**) ATGL activation was analyzed by binding of the fluorescent lipase inhibitor NBD-sn1TG. Cytoplasmic extracts were incubated with fluorescently labeled inhibitor and subjected to SDS-PAGE. NBD-sn1TG-labeled proteins were visualized by a BioRad FX Pro Laserscanner. Data for TGH activity assays are presented as mean ± S.D. and represent at least three independent experiments. (**p* < 0.05, ***p* <0.01, ****p* < 0.001). (**f**) Dose-dependent effect of hCGI-58 on TGH activity of hATGL. The molar ratio ATGL/CGI-58 was determined as described in (**d**).

Fig. 6 shows that CGI-58 affects lipid metabolism as activator of ATGL and appears to represent a major player in cellular lipid metabolism. Regarding the high expression levels of CGI-58 and ATGL in adipose tissue, modulation of the activity of each protein could affect TG and FFA metabolism and hence offer a strategy for the treatment of obesity and related disorders.

### Reference List

1. Bergman, R.N., G.W. Van Citters, S.D. Mittelman, M.K. Dea, M. Hamilton-Wessler, S.P. Kim, and M. Ellmerer. 2001. Central role of the adipocyte in the metabolic syndrome. J Investig Med 49: 119-26.
2. Blaak, E.E. 2003. Fatty acid metabolism in obesity and type 2 diabetes mellitus. Proc Nutr Soc 62: 753-60.
3. Boden, G. and G.I. Shulman. 2002. Free fatty acids in obesity and type 2 diabetes: defining their role in the development of insulin resistance and beta-cell dysfunction. Eur J Clin Invest 32 Suppl 3: 14-23.
4. Arner, P. 2002. Insulin resistance in type 2 diabetes: role of fatty acids. Diabetes Metab Res Rev 18 Suppl 2: S5-9.
5. Collins, S. and R.S. Surwit. 2001. The beta-adrenergic receptors and the control of adipose tissue metabolism and thermogenesis. Recent Prog Horm Res 56: 309-28.
6. Sztalryd, C., G. Xu, H. Dorward, J.T. Tansey, J.A. Contreras, A.R. Kimmel, and C. Londos. 2003. Perilipin A is essential for the translocation of hormone-sensitive lipase during lipolytic activation. J Cell Biol 161: 1093-103.
7. Haemmerle, G., R. Zimmermann, M. Hayn, C. Theussl, G. Waeg, E. Wagner, W. Sattler, T.M. Magin, E.F. Wagner, and R. Zechner. 2002. Hormone-sensitive Lipase Deficiency in Mice Causes Diglyceride Accumulation in Adipose Tissue, Muscle, and Testis. JBiol Chem 277: 4806-4815.
8. Okazaki, H., J. Osuga, Y. Tamura, N. Yahagi, S. Tomita, F. Shionoiri, Y. Iizuka, K. Ohashi, K. Harada, S. Kimura, T. Gotoda, H. Shimano, N. Yamada, and S. Ishibashi. 2002. Lipolysis in the absence of hormone-sensitive lipase: evidence for a common mechanism regulating distinct lipases. Diabetes 51: 3368-75.
9. Wang, S.P., N. Laurin, J. Himms-Hagen, M.A. Rudnicki, E. Levy, M.F. Robert, L. Pan, L. Oligny, and G.A. Mitchell. 2001. The adipose tissue phenotype of hormone-sensitive lipase deficiency in mice. Obes Res 9: 119-28.
10. Zimmermann, R., G. Haemmerle, E.M. Wagner, J.G. Strauss, D. Kratky, and R. Zechner. 2003. Decreased fatty acid esterification compensates for the reduced lipolytic activity in hormone-sensitive lipase-deficient white adipose tissue. J Lipid Res 44: 2089-99.
11. Oskolkova, O.V., R. Saf, E. Zenzmaier, and A. Hermetter. 2003. Fluorescent organophosphonates as inhibitors of microbial lipases. Chem Phys Lipids 125: 103-14.
12. Yeaman, S.J., G.M. Smith, C.A. Jepson, S.L. Wood, and N. Emmison. 1994. The multifunctional role of hormone-sensitive lipase in lipid metabolism. Adv Enzyme Regul 34: 355-70.
13. Wei, S., K. Lai, S. Patel, R. Piantedosi, H. Shen, V. Colantuoni, F.B. Kraemer, and W.S. Blaner. 1997. Retinyl ester hydrolysis and retinol efflux from BFC-1beta adipocytes. J Biol Chem 272: 14159-65.
14. Fredrikson, G., P. Stralfors, N.O. Nilsson, and P. Belfrage. 1981. Hormone-sensitive lipase of rat adipose tissue. Purification and some properties. J Biol Chem 256: 6311-20.
15. Fredrikson, G., H. Tornqvist, and P. Belfrage. 1986. Hormone-sensitive lipase and monoacylglycerol lipase are both required for complete degradation of adipocyte triacylglycerol. Biochim Biophys Acta 876: 288-93.
16. Liu, P., Y. Ying, Y. Zhao, D.I. Mundy, M. Zhu, and R.G. Anderson. 2004. Chinese hamster ovary K2 cell lipid droplets appear to be metabolic organelles involved in membrane traffic. J Biol Chem 279: 3787-92.
17. Baulande, S., F. Lasnier, M. Lucas, and J. Pairault. 2001. Adiponutrin, a transmembrane protein corresponding to a novel dietary- and obesity-linked mRNA specifically expressed in the adipose lineage. J Biol Chem 276: 33336-44.
18. Tatusov, R.L., D.A. Natale, I.V. Garkavtsev, T.A. Tatusova, U.T. Shankavaram, B.S. Rao, B. Kiryutin, M.Y. Galperin, N.D. Fedorova, and E.V. Koonin. 2001. The COG database: new developments in phylogenetic classification of proteins from complete genomes. Nucleic Acids Res 29: 22-8.
19. Bateman, A., L. Coin, R. Durbin, R.D. Finn, V. Hollich, S. Griffiths-Jones, A. Khanna, M. Marshall, S. Moxon, E.L. Sonnhammer, D.J. Studholme, C. Yeats, and S.R. Eddy. 2004. The Pfam protein families database. Nucleic Acids Res 32 Database issue: D138-41.
20. Shewry, P.R. 2003. Tuber storage proteins. Ann Bot (Lond) 91: 755-69.
21. Athenstaedt, K. and G. Daum. 2003. YMR313c/TGL3 encodes a novel triacylglycerol lipase located in lipid particles of Saccharomyces cerevisiae. JBiol Chem 278: 23317-23.
22. Dessen, A., J. Tang, H. Schmidt, M. Stahl, J.D. Clark, J. Seehra, and W.S. Somers. 1999. Crystal structure of human cytosolic phospholipase A2 reveals a novel topology and catalytic mechanism. Cell 97: 349-60.
23. Rydel, T.J., J.M. Williams, E. Krieger, F. Moshiri, W.C. Stallings, S.M. Brown, J.C. Pershing, J.P. Purcell, and M.F. Alibhai. 2003. The crystal structure, mutagenesis, and activity studies reveal that patatin is a lipid acyl hydrolase with a Ser-Asp catalytic dyad. Biochemistry 42: 6696-708.
24. Bernlohr, D.A., M.A. Bolanowski, T.J. Kelly Jr, and M.D. Lane. 1985. Evidence for an increase in transcription of specific mRNAs during differentiation of 3T3-L1 preadipocytes. JBiol Chem 260: 5563-7.
25. Notredame, C., D.G. Higgins, and J. Heringa. 2000. T-Coffee: A novel method for fast and accurate multiple sequence alignment. J Mol Biol 302: 205-17.
26. Thompson, J.D., T.J. Gibson, F. Plewniak, F. Jeanmougin, and D.G. Higgins. 1997. The CLUSTAL_X windows interface: flexible strategies for multiple sequence alignment aided by quality analysis tools. Nucleic Acids Res 25: 4876-82.
27. Bernlohr, D.A., M.A. Bolanowski, T.J. Kelly Jr, and M.D. Lane. 1985. Evidence for an increase in transcription of specific mRNAs during differentiation of 3T3-L1 preadipocytes. J Biol Chem 260: 5563-7.
28. Liu, P., Y. Ying, Y. Zhao, D.I. Mundy, M. Zhu, and R.G. Anderson. 2004. Chinese hamster ovary K2 cell lipid droplets appear to be metabolic organelles involved in membrane traffic. J Biol Chem 279: 3787-92.
29. Oskolkova, O.V., R. Saf, E. Zenzmaier, and A. Hermetter. 2003. Fluorescent organophosphonates as inhibitors of microbial lipases. Chem Phys Lipids 125: 103-14.
30. Holm, C. and T. Osterlund. 1999. Hormone-sensitive lipase and neutral cholesteryl ester lipase. in Lipase and Phospholipase Protocols, M.Doolitttle, K.Reue, Eds. (Humana Press, Totowa, New Jersey) 109, chap.11.

atgtttcc ccgcgagaag acgtggaaca tctcgttcgc
gggctgcggc ttcctcggcg tctactacgt cggcgtggcc tcctgcctcc gcgagcacgc
gcccttcctg gtggccaacg ccacgcacat ctacggcgcc tcggccgggg cgctcacggc
cacggcgctg gtcaccgggg tctgcctggg tgaggctggt gccaagttca ttgaggtatc
taaagaggcc cggaagcggt tcctgggccc cctgcacccc tccttcaacc tggtaaagat
catccgcagt ttcctgctga aggtcctgcc tgctgatagc catgagcatg ccagtgggcg
cctgggcatc tccctgaccc gcgtgtcaga cggcgagaat gtcattatat cccacttcaa
ctccaaggac gagctcatcc aggccaatgt ctgcagcggt ttcatccccg tgtactgtgg
gctcatccct ccctccctcc agggggtgcg ctacgtggat ggtggcattt cagacaacct
gccactctat gagcttaaga acaccatcac agtgtccccc ttctcgggcg agagtgacat
ctgtccgcag gacagctcca ccaacatcca cgagctgcgg gtcaccaaca ccagcatcca
gttcaacctg cgcaacctct accgcctctc caaggccctc ttcccgccgg agcccctggt
gctgcgagag atgtgcaagc agggataccg ggatggcctg cgctttctgc agcggaacgg
cctcctgaac cggcccaacc ccttgctggc gttgcccccc gcccgccccc acggcccaga
ggacaaggac caggcagtgg agagcgccca agcggaggat tactcgcagc tgccgggaga
agatcacatc ctggagcacc tgcccgcccg gctcaatgag gccctgctgg aggcctgcgt
ggagcccacg gacctgctga ccaccctctc caacatgctg cctgtgcgtc tggccacggc
catgatggtg ccctacacgc tgccgctgga gagcgctctg tccttcacca tccgcttgct
ggagtggctg cccgacgttc ccgaggacat ccggtggatg aaggagcaga cgggcagcat
ctgccagtac ctggtgatgc gcgccaagag gaagctgggc aggcacctgc cctccaggct
gccggagcag gtggagctgc gccgcgtcca gtcgctgccg tccgtgccgc tgtcctgcgc
cgcctacaga gaggcactgc ccggctggat gcgcaacaac ctctcgctgg gggacgcgct
ggccaagtgg gaggagtgcc agcgccagct gctgctcggc ctcttctgca ccaacgtggc
cttcccgccc gaagctctgc gcatgcgcgc acccgccgac ccggctcccg cccccgcgga
cccagcatcc ccgcagcacc agctggccgg gcctgccccc ttgctgagca cccctgctcc
cgaggcccgg cccgtgatcg gggccctggg gctgtga

SEQ No. 1

atg
ttcccgaggg agaccaagtg gaacatctca ttcgctggct gcggcttcct cggggtctac
cacattggcg tggcctcctg cctccgtgag cacgcgccct tcctggtggc caacgccact
cacatctacg gagcctcggc aggggcgctc accgccacag cgctggtcac tggggcctgc
ctgggtgaag caggtgccaa cattattgag gtgtccaagg aggcccggaa gcggttcctg
ggtcctctgc atccctcctt caacctggtg aagaccatcc gtggctgtct actaaagacc
ctgcctgctg attgccatga gcgcgccaat ggacgcctgg gcatctccct gactcgtgtt
tcagacggag agaacgtcat catatcccac tttagctcca aggatgagct catccaggcc
aatgtctgca gcacatttat cccggtgtac tgtggcctca ttcctcctac cctccaaggg
gtgcgctatg tggatggcgg catttcagac aacttgccac tttatgagct gaagaatacc
atcacagtgt ccccattctc aggcgagagt gacatctgcc ctcaggacag ctccaccaac
atccacgagc ttcgcgtcac caacaccagc atccagttca accttcgcaa tctctaccgc
ctctcgaagg ctctcttccc gccagagccc atggtcctcc gagagatgtg caaacagggc
tacagagatg gacttcgatt ccttaggagg aatggcctac tgaaccaacc caaccctttg
ctggcactgc cccagttgt cccccaggaa gaggatgcag aggaagctgc tgtggtggag
gagagggctg gagaggagga tcaattgcag ccttatagaa aagatcgaat tctagagcac
ctgcctgcca gactcaatga ggccctgctg gaggcctgtg tggaaccaaa ggacctgatg
accacccttt ccaacatgct accagtgcgc ctggcaacgg ccatgatggt gccctatact
ctgccgctgg agagtgcagt gtccttcacc atccgcttgt tggagtggct gcctgatgtc
cctgaagata tccggtggat gaaagagcag acgggtagca tctgccagta tctggtgatg
agggccaaga ggaaattggg tgaccatctg ccttccagac tgtctgagca ggtggaactg
cgacgtgccc agtctctgcc ctctgtgcca ctgtcttgcg ccacctacag tgaggcccta
cccaactggg tacgaaacaa cctctcactg ggggacgcgc tggccaagtg ggaagaatgc
cagcgtcagc tactgctggg tctcttctgc accaatgtgg ccttcccgcc ggatgccttg
cgcatgcgcg cacctgccag ccccactgcc gcagatcctg ccaccccaca ggatccacct
ggcctcccgc cttgctga

SEQ No. 2

## Claims

1. Method for determining the triglyceride hydrolase activity of a protein comprising a polypeptide strand encoded by the DNA sequence according to SEQ No. 1 in an aqueous sample in presence of hormone sensitive lipase, **characterized in that** alkali metal halogenide is added to the sample in an amount effective to suppress the activity of said hormone sensitive lipase, whereafter the triglyceride hydrolase activity is determined.

2. Method according to claim 1, **characterized in that** said alkali metal halogenide is potassium chloride.

3. Use of CGI-58 (Comparative gene identification 58) as activator of a protein comprising a polypeptide strand encoded by the DNA sequence according to SEQ No. *1 in vitro*.

4. Antibody as inhibitor of the activity of adipose triglyceride lipase encoded by a DNA sequence comprising the sequence according to SEQ ID NO: 1 for use in the treatment of obesity, type 2 diabetes or metabolic syndrome.

## Patentansprüche

1. Verfahren zum Bestimmen der Triglyceridhydrolaseaktivität eines Proteins, das einen Polypeptidstrang umfasst, der von einer DNA-Sequenz gemäß SEQ ID NO:1 codiert wird, in einer wässrigen Probe in Anwesenheit von hormonsensitiver Lipase, **dadurch gekennzeichnet, dass** der Probe Alkalimetallhalogenid in einer Menge hinzugefügt wird, die wirksam ist, um die Aktivität der hormonsensitiven Lipase zu unterdrücken, wonach die Triglyceridhydrolaseaktivität bestimmt wird.

2. Verfahren nach Anspruch 1, **dadurch gekennzeichnet, dass** das Alkalimetallhalogenid Kaliumchlorid ist.

3. *In vitro*-Verwendung von CGI-58 (Comparative Gene Identification 58) als Aktivator eines Proteins, das einen von einer DNA-Sequenz gemäß SEQ ID NO:1 codierten Polypeptidstrang umfasst.

4. Antikörper als Inhibitor der Aktivität von adipöser Triglyceridlipase, die von einer DNA-Sequenz codiert wird, die die Sequenz gemäß SEQ ID NO:1 umfasst, zur Verwendung bei der Behandlung von Adipositas, Typ 2-Diabetes oder metabolischem Syndrom.

## Revendications

1. Procédé pour déterminer l'activité triglycéride hydrolase d'une protéine comprenant un brin polypeptidique codé par la séquence d'ADN selon SEQ ID NO: 1 dans un échantillon aqueux en présence d'une lipase hormono-sensible, **caractérisé en ce qu'**un halogénure métallique alcalin est ajouté à l'échantillon en une quantité efficace pour supprimer l'activité de ladite lipase hormono-sensible, après quoi l'activité triglycéride hydrolase est déterminée.

2. Procédé selon la revendication 1, **caractérisé en ce que** ledit halogénure métallique alcalin est le chlorure de potassium.

3. Utilisation de CGI-58 (Comparative gene identification 58) en tant qu'activateur d'une protéine comprenant un brin polypeptidique codé par la séquence d'ADN selon SEQ ID NO: 1 *in vitro*.

4. Anticorps en tant qu'inhibiteur de l'activité d'une adipose triglycéride lipase codée par une séquence d'ADN comprenant la séquence selon SEQ ID NO: 1 pour une utilisation dans le traitement de l'obésité, du diabète de type 2 ou du syndrome métabolique.
